# EUROPEAN PATENT APPLICATION

(11) **EP 4 632 667 A1**
(43) Date of publication of application: **15.10.2025**
(21) Application number: 24169257.3
(22) Date of filing: 09.04.2024
(51) Int. Cl.: G06T 5/60, G06T 5/70, G06T 5/73

(54) **NEAR TRUE-VIEW MEDICAL VIDEO PROCESSOR**

(71) Applicant: Ambu A/S, 2750 Ballerup (DK)
(72) Inventor: JØRGENSEN, Alex Skovsbo, 3650 Ølstykke (DK); JØRGENSEN, Andreas Härstedt, 2610 Rødovre (DK); GREGERSEN, Søren Kimmer Schou, 4000 Roskilde (DK); LASSEN, Lee Herluf Lund, 2760 Måløv (DK)
(74) Representative: AWA Denmark A/S

(57) **Abstract**

A method to generate near real-view images and an image processor configured to execute the method. The method includes, by an image processing circuit (100) connected to a videoscope (20'): receiving an image (202a) from the videoscope (20'); processing a source image (204a) corresponding to the image (202a) with a single denoising and feature/edge detection trained network (SDDTT) (124), the SDDTT outputting, in a single pass, a denoise map (206a) and an edge map (206b); denoising the source image (204a) with the noise map (206a) to produce a denoised image (208a); gamma-correcting the denoised image to produce a gamma-corrected image (210a); and sharpening the gamma-corrected denoised image with the edge map (208b) to produce the near real-view image (212a).

## Description

### CROSS-REFERENCE TO RELATED APPLICATIONS

None.

### TECHNICAL FIELD

The disclosure relates to a video processor operable to output images obtained with a videoscope. More particularly, the disclosure relates to a video processor operable to receives images from one or more videoscopes and output a true-view video stream corresponding to the images for presentation with a display.

### BACKGROUND

Video processors comprise a video processing circuit, or circuits, operable to receive image data from a videoscope and to output video signals for presentation of images with a display. The display can be integrated with the video processor or may be a separate part communicatively coupled to the video processor. Video processors with integrated displays offer many advantages and conveniences in many settings including in the field, emergency response vehicles and hospitals. However, upgrading the display integrated with the video processor might not be technically or economically feasible. Video processors with separate displays can take advantage of existing investments in displays and also of new display technologies.

Images generated with image sensors inside body cavities can be noisy and exhibit varying pixel intensities depending on the image sensor used, the location and quality of light emitters used to illuminate target areas, the proximity of the tip of the videoscope to tissue, the amount of moisture present, tip occlusion, and other factors. For example, if the tip of the videoscope is adjacent to one side of a lumen, the image will have higher intensity pixels on the adjacent side and lower intensity pixels on the opposite side. Lower intensities can result in increased noise while overexposure can be apparent on the adjacent side. Moisture and occlusion can also limit lighting and thus increase noise.

A videoscope is a device comprising an image sensor at a distal end thereof configured to obtain images of views reflected from objects or tissue positioned distally or laterally of the distal end of the videoscope. Medical videoscopes are configured to obtain images of internal views of the patient and include endoscopes, video laryngoscopes, video endotracheal tubes, and any other medical device configured for insertion into the patient that comprises an image sensor at its distal end. The term "patient" herein includes humans and animals. Some, but not all, videoscopes include working channels.

A known problem with image processing is the conflicting effects of denoising and sharpening. In known image processing techniques, excessive sharpening increases noise and excessive denoising limits sharpness. The characteristics of the videoscope can also contribute noise.

Medical videoscopes are made for various procedures and may have different technical characteristics suited for the procedure they are designed to perform, based on the age of the device, or for other reasons. The technical characteristics, or technology, may comprise the type of image sensor included with the videoscope, whether the videoscope includes on-board data processing capabilities, and whether the videoscope includes additional sensors which provide information to a video processor, potentially including more than one image sensor. The type of image sensor may provide different capabilities, including resolution and various controls such as image inversion, image rotation, contrast, and exposure. An endoscope, both reusable and disposable (i.e. single-use), is a species of a videoscope. Endoscopes include procedure-specialized devices, for example arthroscopes, bronchoscopes, cholangioscopes, colonoscopes, cystoscopes, duodenoscopes, gastroscopes, laparoscopes, ureteroscopes, and others.

It is desirable to improve image quality by improving the technologies used to manufacture videoscopes. However, improved technologies often increase costs. When the videoscopes are configured to be disposed after a single use on a patient, increased costs are undesirable. Additionally, it is not possible to improve the image quality of already manufactured videoscopes, therefore there may be a significant supply-chain pipeline of devices which would benefit from image quality improvements resulting from improved image processing techniques.

### SUMMARY

The present disclosure provides solutions which at least improve the solutions of the prior art. In some aspects of the disclosure, solutions are provided to present on a display images that are more closely representative of real-views than those obtained with prior-art devices. This is achieved by processing source images with a single denoising and feature/edge detection trained network (SDDTT) to generate near real-view images. The source images are processed in real-time. However, the images can also be recorded and processed off-line.

An object of the invention disclosed herein is to produce near real-view images. An advantage of doing so is that images of quality approaching that of higher resolution image sensors can be produced with lower resolution image sensors, which are typically less expensive than higher resolution sensors. This enables manufacture of lower cost videoscopes, in particular single-use videoscopes. Another advantage is that the processing cost of low resolution images, including functional processing and near real-view processing, may be less than the processing cost of functional processing of high resolution images. Processing costs include processor cycles, movements of data in memory, and other data processing steps that, generally, translate to time and power usage, time being very relevant in the use of devices under time constraints (e.g. frame-rate) and particularly relevant to battery-powered devices, such as portable video processors. Processing costs may drive the cost of the hardware used to process images, for example in the form of additional memory and faster processors, therefore lower processing costs may enable the manufacture of lower cost video processors and/or the addition of functions without increasing the cost of the video processor.

As used herein, "near real-view images" are images processed with the SDDTT to increase the relative quality of the images as compared to images generated by prior-art devices. Additionally, it should be understood that near real-view images can have different quality levels depending on the quality/resolution of the images provided by the videoscope. By contrast, functional processing refers to the functions performed by the video processor to provide a particular "product". Examples of functional processing include image-based object detection, such as to identify landmarks, encryption and decryption, navigation, and the like. Another way to think about near real-view images is by recognizing that a real-view is a view of tissue or substance in the field-of-view of the image sensor. In the process capturing the real-view, the image sensor captures artifacts such as Bayer filter effects and lens distortion. Furthermore, the captured image will include illumination artifacts arising from illumination distribution and noise. Noise may be more prevalent in poorly illuminated areas of the image. As the captured image is transmitted to an image processor, the captured image may incorporate electrical or electromagnetic noise/ artifacts. Perfect removal of all the artifacts would convert an image into a real-view image.

A first aspect of the invention is to provide a method for generating near real-view images. A second aspect of the invention is to provide a video processor that implements the method according to the first aspect. A third aspect of the invention is to provide a visualization system that implements the method according to the first aspect.

In an embodiment according to the first aspect, a method to generate near real-view images is provided, the method comprising: by an image processing circuit connected to a videoscope: receiving an image from the videoscope; processing a source image corresponding to the image with a single denoising and feature/edge detection trained network (SDDTT), the SDDTT outputting, in a single pass, a denoise map and an edge map; denoising the source image with the noise map to produce a denoised image; gamma-correcting the denoised image to produce a gamma-corrected image; and sharpening the gamma-corrected denoised image with the edge map to produce the near real-view image.

In a variation of the first embodiment, the method further comprises demosaicing the image before processing the source image with the SDDTT. In one example, after said denoising and before said gamma-correcting the demosaiced image, the method comprises color-converting the demosaiced image to a YUV color space to form a YUV image, gamma-correcting the YUV image, and after said gamma-correcting, sharpening a Y-channel of the YUV image. Sharpening the Y-channel may reduce processing speed vis a vis sharpening three channels of an RGB image.

In a variation of the first embodiment, the source image processed by the SDDTT is a raw image, and the method further comprises, after said denoising and before said gamma-correcting, demosaicing the raw image.

In a further variation of the first embodiment, the videoscope comprises an image sensor, and the method further comprises, by the image processing circuit, determining a type or a model of the image sensor. In one example in which the image processing circuit comprises two or more trained networks, the method further comprises, by the image processing circuit, selecting the SDDTT from the two or more trained networks, wherein each of the two or more trained networks was trained with images collected with a different image sensor type.

In a yet further variation of the first embodiment, wherein the videoscope comprises a first image sensor of an image sensor type, the method further comprises capturing a first plurality of images with a second image sensor of the image sensor type at a first image sensor gain; capturing a second plurality of images with the second image sensor at a second image sensor gain, the second image sensor gain being different than the first image sensor gain; providing the first image sensor gain and the first plurality of images to a single denoising and feature/edge detection network; processing the first plurality of images with the single denoising and feature/edge detection network to train the single denoising and feature/edge detection network; providing the second image sensor gain and the second plurality of images to the single denoising and feature/edge detection network; and processing the second plurality of images with the single denoising and feature/edge detection network to further train the single denoising and feature/edge detection network and form the SDDTT.

In another variation of the first embodiment, the method further comprises, before processing the source image with the SDDTT, reducing fixed pattern noise in an image corresponding to the image. The image with the fixed pattern noise may be the image received from the videoscope or an image based on the image received from the videoscope upon which some pre-processing was performed before the fixed pattern noise is reduced.

In still another variation of the first embodiment, the SDDTT comprises a decoder and an encoder, the decoder comprising four decoder blocks, each decoder block comprising a convolution layer, an activation layer, and a subsampling layer, each of the convolution layers comprising trainable parameters, and wherein the encoder comprises encoder blocks including a first encoder block, a second encoder block following the first encoder block, and a third encoder block following the second encoder block, each of the encoder blocks comprising a convolution layer, an activation layer, an upsampling layer, and a concatenation layer, wherein the SDDTT further comprises, after the third encoder block, a first specialized block comprising a convolution layer and being configured to output the noise map, and wherein the SDDTT further comprises a second specialized block comprising a convolution layer and being configured to output the edge map.

The following three examples of the present variation provide optional performance improvements balancing speed and quality. In the first example, the encoder further comprises a fourth encoder block following the third encoder block, wherein the first specialized block receives an output from the concatenation layer of the fourth encoder block, and wherein the second specialized block receives an output from the upsampling layer of the fourth encoder block. By following it is meant that the layer or block processes images after the layer/block it follows. The following layer/block may receive the output (image) of the preceding layer/block directly or via an intermediary layer/block.

In the second example, the encoder further comprises a fourth encoder block following the third encoder block, wherein the first specialized block receives an output from the concatenation layer of the fourth encoder block, and wherein the second specialized block receives an output from the concatenation layer of the third encoder block.

In the third example, the encoder further comprises a downsampling layer and a fourth concatenation layer, wherein the fourth concatenation layer follows the downsampling layer and the third encoder block, wherein the first specialized block receives an output from the fourth concatenation layer and comprises an upsampling layer following the convolution layer, and wherein the second specialized block receives the output from the fourth concatenation layer and comprises an upsampling layer following the convolution layer.

In still another variation of the first embodiment, which may include one or more of the aforementioned variations and examples, the image processing circuit comprises a controller and non-volatile memory, the non-volatile memory having embedded therein the SDDTT, and the controller comprising a central processing unit and a graphics processing unit.

In an embodiment according to the second aspect, a video processor comprises a controller comprising the image processing circuit, the image processing circuit including a non-volatile memory having embedded therein processing instructions including the SDDTT, the processing instructions being configured to implement the method according to the first aspect and any of the aforementioned variations and examples thereof.

In an embodiment according to the third aspect, a visualization system comprises a videoscope, a display, and the video processor according to the second aspect, wherein the processing instructions are configured to present with the display images corresponding to the near real-view image.

One or more of these objects may be met by aspects of the present invention described in the foregoing and following embodiments, variations and examples thereof.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

The disclosure will be described in more detail below with reference to the following figures. The figures illustrate embodiments, variations and examples of the invention to facilitate the understanding of a person of ordinary skill in the art and are not to be construed as limiting the scope of the invention.
FIG. 1 is a schematic illustration of a visualization system;
FIG. 2 is a schematic illustration of an embodiment of a video processor;
FIG. 3 is a block diagram of an embodiment of a video processor circuit;
FIG. 4 is a flowchart of an embodiment of a method to create near real-view images;
FIG. 5 is a flowchart of an embodiment of a method to train a neural network;
FIG. 6 is a schematic depiction of an embodiment of a neural network architecture;
FIG. 7 is a flowchart of a variation of the embodiment of the method to create near real-view images depicted in FIG. 4;
FIG. 8 is a flowchart of another variation of the embodiment of the method to create near real-view images depicted in FIG. 4;
FIG. 9 is a flowchart of a further variation of the embodiment of the method to create near real-view images depicted in FIG. 4; and
FIGS. 10-12 are block diagrams of embodiments of convolutional neural networks used to generate the near real-view images according to the methods of FIGS. 4 and 7-9.

### DETAILED DESCRIPTION OF SELECTED EMBODIMENTS

In the following description, for purposes of explanation, specific details are set forth in order to provide an understanding of the invention. It will be apparent, however, to one skilled in the art that the invention can be practiced without all these details. Furthermore, one skilled in the art will recognize that embodiments of the present invention, described below, may be implemented in a variety of ways, such as a process, an apparatus, a system, a device, or a method on a tangible computer-readable medium.

Components, or modules, shown in diagrams are illustrative of exemplary embodiments of the invention and are meant to avoid obscuring the invention. It shall also be understood that throughout this discussion components may be described as separate functional units, which may comprise sub-units, but those skilled in the art will recognize that various components, or portions thereof, may be divided into separate components or may be integrated together, including integrated within a single system or component. It should be noted that functions or operations discussed herein may be implemented as components. Components may be implemented in software, hardware, or a combination thereof.

Furthermore, connections between components or systems within the figures are not intended to be limited to direct connections. Rather, data between these components may be modified, re-formatted, or otherwise changed by intermediary components. Also, additional or fewer connections may be used. It shall also be noted that the terms "coupled," "connected," or "communicatively coupled" shall be understood to include direct connections, indirect connections through one or more intermediary devices, and wireless connections.

The following descriptions concern image processing of images in multiple steps, referred to as image pipelines. Image pipelines may include processing blocks, some of which may be prior-art and/or optional. In this context, the term "corresponding" is indicative of an image containing information from a preceding image, where the preceding image may have been processed in different ways. Thus, the format of the corresponding image may differ from the format of the preceding image and the pixels of the corresponding image may differ from the pixels of the preceding image, due to a transformation of the image, while retaining the relevant original image content. For example, fixed noise removal retains image features, which is the relevant image content. For another example, a raw (preceding) image may be demosaiced into an RGB (corresponding) image, and the RGB (preceding) image may be transformed to a YUV (corresponding) image. The YUV image, therefore, corresponds to the raw and the RGB images since it includes their content, albeit transformed. In another example, a raw bayer pattern image of, for example, 800x800 pixels (preceding image), can be divided into 400x400x4 channels (channels are blue-, green-, green-, and red pixels only, a corresponding image). The term "corresponding" is therefore use to allow for intermediate processing steps.

Reference in the specification to "one embodiment," "preferred embodiment," "an embodiment," or "embodiments" means that a particular feature, structure, characteristic, or function described in connection with the embodiment is included in at least one embodiment of the invention and may be in more than one embodiment. Also, the appearances of the above-noted phrases in various places in the specification are not necessarily all referring to the same embodiment or embodiments. Furthermore, the use of certain terms in various places in the specification is for illustration and should not be construed as limiting. Any headings used herein are for organizational purposes only and shall not be used to limit the scope of the description or the claims.

Furthermore, it shall be noted that: (1) certain steps may optionally be performed; (2) steps may not be limited to the specific order set forth herein; (3) certain steps may be performed in different orders; and (4) certain steps may be done concurrently.

The disclosures of the following U.S. patents are incorporated herein by reference in their entirety: U.S. Patent No. 4,774,565, U.S. Patent No. 11,096,553, U.S. Patent No. 11,166,624, 11,328,390, and U.S. Patent No. 11,730,341.

As indicated above, one advantage of the invention is the removal of noise. The removal of noise, in particular image sensor generated noise, allows a video processor according to embodiments of the invention to more effectively identify and highlight edges and texture, thereby enabling the video processor to cause the presentation of near real-view images and/or video streams to the user. Removal of noise by other techniques, such as averaging, can remove too much information from the images, potentially removing image features of interest, in particular edges of smaller tissue structures.

To generate the near real-view images, the SDDTT, also referred to as a trained model, generates a denoise map and an edge map (i.e. edge and texture). One of the training challenges is to train without patient data. Therefore, the residual noise map is predicted to ensure that the network learns to detect noise independent of the content in the scene. The denoise map is subtracted from a source image to remove noise and then the edge map is added to the denoised image to generate the near real-view image. Obtaining both the denoise map and the edge map from a single neural network, potentially in a single pass, reduces computational time compared to using two separate neural networks, one for each task. Perhaps more importantly, using a neural network with both a denoise output block and an edge output block decorrelates the edge/texture information from the noise to produce better results compared to using two neural networks. As is known in prior art techniques, denoising and sharpening work against each other, therefore reducing noise reduces sharpness and increasing sharpness increases noise. The single trained neural network thus produces the synergistic result of simultaneously reducing noise and increasing sharpness, potentially in one pass per image. Another synergistic result is obtained by processing images together with an image gain value. By training the model with images and image gain values, the trained model can better distinguish the noise in the images and thereby produce even better noise maps than if the gain value were not provided as an input.

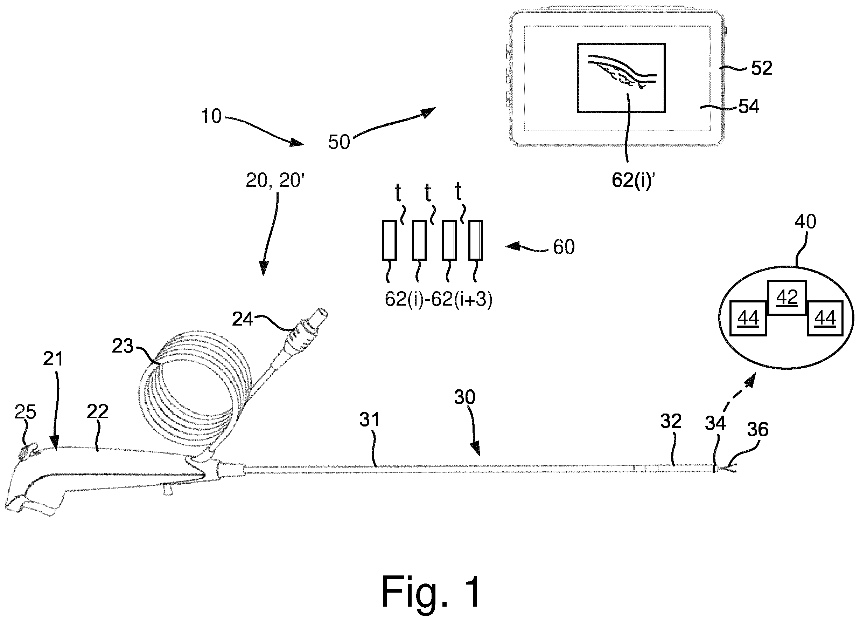

FIG. 1 provides overall context by illustrating a visualization system 10 comprising a videoscope 20', illustratively an endoscope 20, a video processor 50, and a stream of images 60 (each image identified as 62(i) to 62(i+3)) transmitted from the videoscope to the video processor 50, where the images are processed with the trained model to generate the near real-view images. A near real-view image 62(i)' is obtained by the disclosed method from an image 62(i) and presented with the video processor 50.

The endoscope 20 comprises a handle 21 comprising a housing 22 and a steering actuator 25. An endoscope cable 23 includes a connector 24 that is receivable by a connector receptacle of the video processor 50 to establish electronic communications between the video processor 50 and the endoscope 20. The endoscope 20 also comprises an insertion cord 30 including an insertion tube 31, a bending section 32, and a distal tip 34. In the present embodiment the endoscope 20 comprises a working channel. A tool 36 is shown extending through the insertion cord 30, through the working channel, and out the distal tip 34. The distal tip 34 includes a camera assembly 40 including an image sensor 42 and light emitters 44. The light emitters may be light emitting diodes (LEDs) or distal ends of optical fibers. In this embodiment the video processor 50 comprises a housing 52 and an optional display 54.

By "live view" it is meant that images or video are received by the video processor 50 from the videoscope 20' and presented in substantially real-time. As shown, the video stream 60 comprises four frames 60(i)-60(i+3), spaced in time by a time period t corresponding to the frame-rate at which the video stream 60 was captured by the image sensor. Consequently, image processing performed by the video processor 50 should be fast enough to enable presentation of a live view.

By "real-time" it is meant that the image processor 50 processes the images/video generated by the videoscope 20' while the videoscope 20' generates them with minimal (in the order of milliseconds or less than 6 frames at 30 fps, preferably 3 or less frames) latency so that the physician observing the live view can rely on the view being representative of the current position of the videoscope.

As mentioned above, an endoscope is a species of a videoscope, which is a device comprising an image sensor at a distal end thereof configured to obtain images of views reflected from objects or tissue positioned distally or laterally of the distal end of the videoscope. Medical videoscopes include endoscopes, video laryngoscopes, video endotracheal tubes. The term "patient" herein includes humans and animals. Some, but not all, videoscopes include working channels. Endoscopes, both reusable and disposable (i.e. single-use), include procedure-specialized endoscopes, for example arthroscopes, bronchoscopes, cholangioscopes, colonoscopes, cystoscopes, duodenoscopes, gastroscopes, laparoscopes, ureteroscopes, and others.

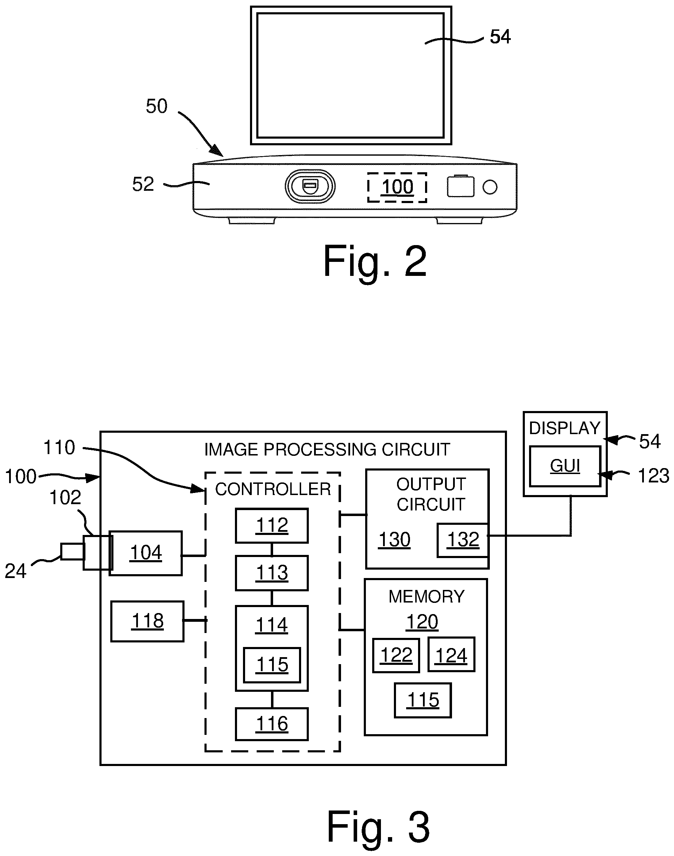

FIG. 2 presents another embodiment of the video processor 50. In this embodiment, the video processor 50 comprises the housing 52. However, the display 54 is not integrated with the video processor 50. Instead, the display 54 is communicatively coupled to the video processor 50. In both embodiments, the housing 52 protects the circuits that perform the functions described below. In other embodiments, the circuits can be integrated in a housing of another apparatus, such as a computer or a computer network. The video processor 50 comprises an image processing circuit 100. Preferably, the video processor 50 is portable, meaning that it can be picked-up and held by a user.

Variations of the video processor 50 can be provided with various features of the video processor 50 but including or excluding other features. For example, it might not be desirable to provide a display with a touch screen, or it might be desirable to omit a display altogether. Omission of the display might be beneficial to take advantage of evolving display technologies which improve resolution and reduce cost. Provision of exchangeable videoscope interfaces allows for adoption of evolving image sensor and videoscope technologies, thus use of existing or future-developed external displays could allow presentation of higher resolution or otherwise improved video. Use of external displays could also leverage existing capital investments. The video processor 50 is configured to present a live view corresponding to the images captured by the image sensor 42.

FIG. 3 is a block diagram of an embodiment of the image processing circuit 100. The image processing circuit 100 depicted in FIG. 3 comprises a cable socket 102, a videoscope interface 104, a controller 110, a memory 120, and a video output board 130. One or more rigid circuit board parts may be provided to mount some or all the electronic parts, including the controller 110, the memory 120, and the video output board 130. The image processing circuit 100 interconnects the videoscope interface 104 with the controller 110, the memory 120, a user interface 118, and the video output board 130 in any manner known in the art.

The videoscope interface 104 may include a cable socket 102 and circuits to compatibilize, e.g. pre-process, the signals from the image sensor 42 to what the controller 110 expects to receive, in terms of image format, for example. Thus, a particular type of videoscope is matched with a corresponding videoscope interface and the video processor 50 can thus enable use of different videoscope technologies. The videoscope interfaces may also include isolation amplifiers to electrically isolate the video signal from the videoscope, and a power output connector to provide power to the videoscope for the image sensor and the LEDs. The videoscope interfaces may also include a serial to parallel converter circuit to deserialize the video signals of endoscopes that generate serial signals, for example serial video signals. The videoscope interfaces may also include analog to digital converters to digitize analog signals generated by the image sensor. In other words, the videoscope interfaces may be configured to receive analog or digital image signals. The videoscope interfaces may also comprise wireless transceivers to receive the image signals from the videoscope wirelessly. The videoscope interfaces may be removable so that various videoscopes may be used by inserting corresponding videoscope interfaces in the video processor. Multiple videoscope interfaces 104 may be provided to enable connections to multiple videoscopes. In some variations, the videoscope and the videoscope interfaces comprise wireless transceivers. In such variations the cable 23, the connector 24, and the connector receptacle can be omitted.

The videoscope interfaces may also include configuration connectors (as part of the cable socket) to output image sensor configuration parameters, such as image inversion, clock, shutter speed etc., and to receive configuration information. An I²C protocol may be used to read and/or control the image sensor over data wires extending between the image sensor and the image processor. The data wires do not transmit images, the images (image signals) are transmitted over image wires. If the image sensor has four connectors, for four wires, one of the wires is a data wire, another is an image signal wire, and the remaining two are ground and power wires. The image processor may read, for example, an image sensor gain indicative of the amount of gain the image sensor automatically uses to compensate for low light conditions. The image processor may write, for example, an image sensor gain indicative of the gain the image processor wants the image sensor to apply. The image processor may, for example, seek to lower the gain to obtain a darker image and prevent overexposing areas of the image. The gain, typically between values 1-16, or 1-4, depending on the sensor, can be set automatically by the image sensor or can be controlled by the image processor via configuration signals sent to the image sensor over the data channel.

The amount of gain also affects noise. Higher gain values have lower signal-to-noise ratios. It is therefore desirable to minimize gain. When training the neural network it is possible to provide the neural network with a gain value corresponding to the gain applied by the image sensor when the image was captured. In this manner the neural network can correlate noise and gain, thereby gain is a parameter that can improve training results and thus the effectiveness of the trained model at decorrelating noise and image features.

When the videoscope is connected to the image processor, based on the sensor specification of the particular image sensor, the image processor will determine the gain range. The image processor may set a gain value, which may be a midrange fixed gain. The amount of gain also affects noise, with higher gain values producing lower signal-to-noise ratios. It is therefore desirable to set the gain value at the lowest value that still produces sufficiently bright images. The image processor may read a sensor identifier (ID) and based on the sensor ID determine, by reading from a table in memory, for example, the gain range of the connected image sensor. An image sensor may also maintain gain parameters in registers and in that case the image processor may read the gain parameters directly from the image sensor.

An exposure value can be used in a similar manner. Even better results may be obtained using both exposure and gain, since the more characterization of images that the model receives the better it can be trained. Current image sensors for endoscopes typically have fixed aperture and ISO (International Organization for Standardization) sensitivity, therefore the exposure triangle, which comprises aperture, shutter speed, and ISO sensitivity, can be determined by the shutter speed. Some image sensors have an auto exposure control (AEC) which comprises exposure and gain. AEC may thus be used as well as a training and processing input. For training and in use, the image processing circuit can read the value determined by the image sensor via the AEC or use a gain value transmitted to the image sensor.

As image sensors evolve it may be possible to incorporate ISO sensitivity in the same manner. In sum, image sensor parameters that are set by the camera and can be read, or parameters that can be set, which parameters control the brightness of the image (and defacto noise) can be used as network inputs to improve performance.

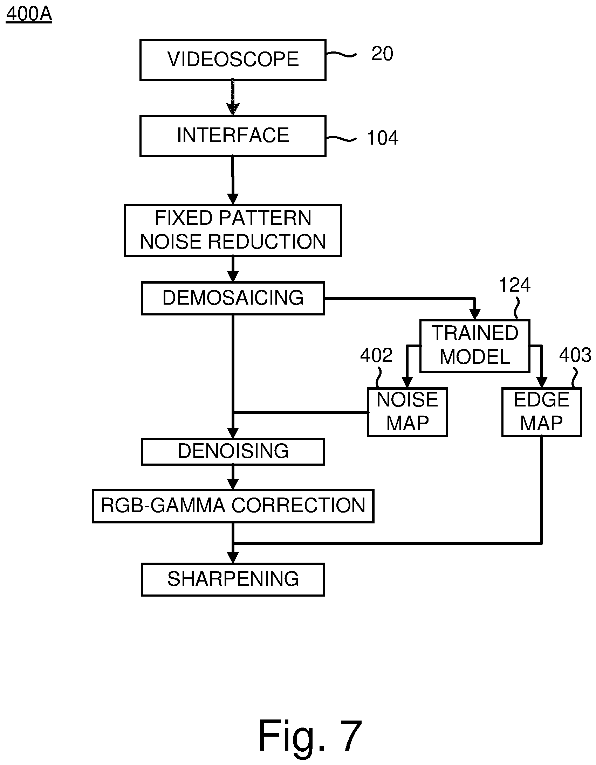

As indicated above, a data channel can be used to transmit configuration parameters from the image processor to the image sensor. The same data channel can be used to read registers of the image sensor and thus obtain gain, exposure, AEC and other parameter values. Once the image processor determines those values, by setting them or by reading them, the image processor can embed the values in each image received from the videoscope. This may be done, for example, by substituting data in specific pixel locations with parameter values. Image corners are useful for this purpose, but other locations may be used also. As discussed below with reference to FIG. 7, embedding image sensor parameter values in each image has the advantage that the values can be read within the pipeline without requiring a separate timing and control scheme to provide the information at the right time, and this approach thus simplifies the controller architecture.

Different image sensors may be comprised in different videoscopes. The image processing techniques described below may be more effective if the SDDTT is trained using images obtained from an image sensor of the same type, preferably having the same resolution, as the source images processed subsequently by the SDDTT. It is therefore desirable for the video processor to recognize the type of image sensor of the videoscope and, potentially, to select an SDDTT trained with the same or substantially the same sensor. To this end the connector 24 and the connector receptacle may be color-coded, permitting the video processor to select a SDDTT based on which videoscope interface provides the image signals. Other ways to identify the technology of the videoscope or image sensor include the image data format, a model number transmitted with the image signals, variations in the type of connector used, etc. Examples of color-coding schemes and other technology matching schemes based on shape and indicators are disclosed in commonly-owned U.S. Patent No. 11,166,624. An EEPROM may be provided in the videoscope, the EEPROM having embedded therein an identification number of the videoscope, which may be used by the image processor to look-up the technology and image sensor of the videoscope in a preconfigured registry provided in the video processor.

As used herein, the term "controller" means a device or devices capable of processing instructions. A controller typically converts coded instructions into timing and control signals that direct the operation of the other components of the device or system, such as memory, arithmetic logic unit, input and output devices, etc. Examples of controllers include complex programmable logic devices (CPLD), central processing units (CPU), graphic processing units (GPU), field programmable gate arrays (FPGAs), a master control unit (MCU) etc. A controller may be a single integrated circuit part or may comprise more than one integrated circuit part. For example, a controller may comprise a combination of a CPU and an FPGA, or a combination of a CPU, a GPU, and an FPGA. The FPGA and GPU may perform graphics processing functions while the MCU performs, as is known, timing and control functions. If the controller comprises more than one integrated circuit part, the integrated circuit parts are linked in a supervised or a distributed manner. For example, a primary integrated circuit part can instruct other integrated circuit parts to execute tasks programmed for the other integrated circuit parts. Alternatively, the other integrated circuit parts may execute their functions independently.

The controller 110 may comprise a CPU 112, an MCU 113, an FPGA 114, and a GPU 116. The CPU 112 performs functions typically performed by CPUs. In the present embodiment a GPU 116 is desired due to the processing requirements of the trained model, including the image processing and the time available to perform the image processing. FPGAs process data very fast compared to other non-volatile memory/instruction combinations and are re-programmable. Therefore, FPGAs facilitate presentation of the live view of the images captured by the endoscope in real-time with minimal latency so that the physician observing the live view can take immediate actions even in emergency situations. The FPGA may process the raw image data generated by the videoscope by performing known optimization functions such as white balance, denoising and the like. The FPGA is optionally provided because it is capable of rapid power-up (i.e. short boot-up time) and thus is useful in emergency situations. Additionally, processing by the trained model may be initiated while the FPGA is already providing a live view, albeit not a near real-view until the trained model is processing images. As technology evolves, the functionality of the FPGA 114 may be performed without the FPGA 114. The video processor 50 is therefore not limited to the precise packaged integrated circuits described with reference to FIG. 3 but can be constructed to take advantage of design and cost targets and future video processing technologies. For example, faster/more costly memory may be used to increase graphics processing speed. Graphics processing may be provided in the FPGA or a processor that incorporates graphics processing logic, such as a GPU. The image processing circuit 100 comprises processing instructions 115 which may be embedded in the FPGA 114, in the memory 120, or in other memory, such as device embedded memory.

A user interface 118 may be provided in the image processing circuit 100. The user interface 118 may comprise a wireless interface operable to receive user inputs via a mouse, keyboard, or other physical user input devices. Example wireless interfaces include Bluetooth and Zigbee controllers. The user interface 118 may comprise a USB port to receive a USB connector of a wired user input device or a USB wireless interface operable to communicate wirelessly with the mouse, keyboard, and/or other physical user input devices including outputs from the touch display 54. Thus, the video processor 50 provides flexibility in receiving user inputs via various user input devices as is known in the art.

The processing instructions 115 embedded in the memory 120 may comprise a graphical user interface (GUI) logic 122, an SDDTT 124, and processing instructions to perform other image processing steps described below. The memory 120 may comprise multiple interconnected circuits, including a memory circuit embedded in the controller 110, a memory integrated circuit connected to the controller 110, a hard-drive connected to the controller 110, and any other devices operable to store data and communicate with the controller 110. Memory includes volatile and non-volatile memory.

The term "logic" as used herein includes software and/or firmware executing on one or more programmable processing devices, application-specific integrated circuits, field-programmable gate arrays, digital signal processors, hardwired logic, or combinations thereof. Therefore, in accordance with the embodiments, various logic may be implemented in any appropriate fashion and would remain in accordance with the embodiments herein disclosed. Logic may comprise processing instructions embedded in non-transitory machine-readable media (e.g. memory).

The GUI logic 122 comprises processing instructions to generate a GUI 123 presented with or by the video processor 50. The GUI can be responsive to user inputs received via the touch screen or other user inputs. The controller 110 receives video (image data), potentially pre-processed by the FPGA, and outputs video signals incorporating the GUI and image data via an output port 132 of the video output board 130, optionally a High-Definition Multimedia Interface (HDMI) port. In one variation, the controller 110 receives raw, unprocessed or pre-processed image data, and converts the image data by generating three (RGB) color channels. The images provided to the SDDTT are referred to as "source images". The RGB images may comprise source images, in some examples. The controller 110 processes the source images with the SDDTT 124 to generate near real-view images, and outputs RGB image data comprising real-view images for presentation with a display. The GUI 123 may comprise a record button that can be toggled to record a clip of the live video. The GUI 123 may comprise additional buttons to provide additional functionality. Thus, the controller 110 causes presentation of the live view of the image 62(i)' with the GUI 123.

In one embodiment, the FPGA 114 is programmed with processing instructions to pre-process image data. Multiple image pipelines may be provided to handle the different image sensor types. Pre-processing may comprise applying known techniques before processing the images with the trained model. Example prior-art pre-processing techniques include fixed pattern noise reduction, green imbalance correction, shading, color correction, etc. Each technique may be applied as a block of code. A mobile industry processor interface (MIPI) bridge may be provided to connect the CPU to the FPGA. Generally, the code blocks remove artifacts caused by the image sensor, cables, and videoscope optics.

In another embodiment, the processing instructions 115 embedded in the memory 120 are configured to pre-process the image data. The processing instructions 115 embedded in the memory 120 may be configured to process image data from different image sensor types. Multiple image pipelines may be provided to handle the different image sensor types. Pre-processing may comprise applying known techniques before processing the images with the trained model. Example prior-art pre-processing techniques include fixed pattern noise reduction, green imbalance correction, shading, color correction, etc. Each technique may be applied as a block of code. Generally, the code blocks remove artifacts caused by the image sensor, cables, and videoscope optics. The processing instructions 115 embedded in the memory 120 may be configured to cause the GPU 116 to pre-process the images instead of the FPGA 114, although the FPGA 114 may perform some image processing as well.

Fixed pattern noise is generally caused by long analog transmission distances between the image sensor and the image processor. It can also come from imperfections in the silicone of the image sensor, including of image sensors with digital image output. An I²C protocol may connect the CPU to the MCU, which provides timing and control signals, as is known in the art. The CPU provides the GUI data to the FPGA where it is combined with the pre-processed image data to generate low voltage differential signals (LVDS) signals to transmit to images to the output interface board.

In the present embodiment, the FPGA may perform exposure and gain control of the image sensor to maintain a desired image brightness. The FPGA and the image sensor may communicate over a SCCB channel or any other known data channel. The image sensor of the videoscope may have a plurality of registers in which data is stored, the data comprising, for example, the sensor ID, saturated pixel limits, gain, gain offset, color weight registers, image gain limits, image color limits, virtual exposure limits, etc. The registers are designed by the manufacturer of the image sensor. The registers may be comprised in the EEPROM, as mentioned above. The MCU, which is communicatively connected to the EEPROM, may read the identification number in the EEPROM and then find and execute the configuration files for that videoscope.

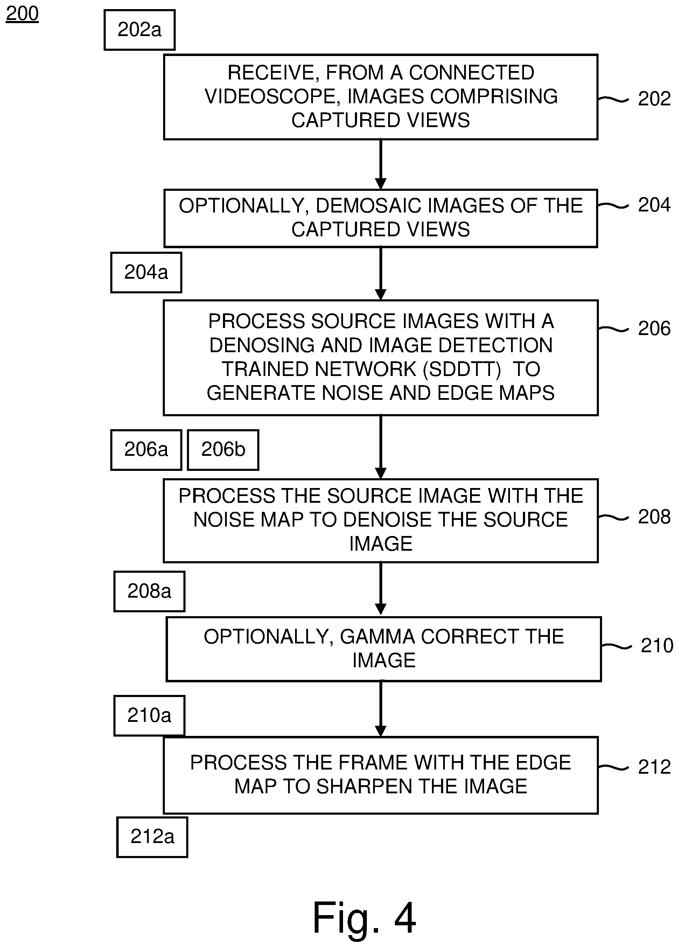

Having described embodiments and variations of video processor hardware, attention will now turn to an embodiment of a method implemented by a video processor according to said embodiments and variations thereof, to cause presentation of a live view comprising near real-view images corresponding to images obtained from a videoscope connected to the video processor. The video processor comprises a videoscope interface 104 configured to receive image data from a videoscope 20', a controller 110; and non-volatile memory 120, the non-volatile memory 120 having embedded therein processing instructions including the SDDTT 124, the SDDTT comprising a denoising output block and a feature detection output block, the processing instructions being executable by the controller 110 to perform the following operations, described with reference to a flowchart 200 shown in FIG. 4. In FIG. 4, labels with alphanumeric characters represent images at various stages of processing.

At 202, the video processor receives image signals comprising images 202a of captured views from a connected videoscope. The term "captured view" is used to distinguish from the term "image," an image referring to a collection of signals and/or pixel data which as a group comprise information depicting a view seen by the videoscope and converted by the image sensor into the image. The image signals may be analog signals or digital signals, e.g. digital data, and may be referred to as videoscope images. The video processor may receive the image signals, transmitted in real time by the videoscope, via the videoscope interface. The videoscope interface may pre-process the signals. For example, the videoscope interface may digitize the analog signals or pad images to a size corresponding to a size optimal for a connected display. The videoscope interface makes available the images comprising the captured views to other components of the video processor, for example by placing the images in a buffer or other volatile memory.

Optionally, the Bayer images may be divided into their four color channels. For example, a raw bayer pattern image of 800x800x1 pixels (preceding image, 1 channel) can be divided into a corresponding image of 400x400x4 pixels (channels are blue-, green-, green- and red pixels only, 4 channels). Each of the four channels comprises ¼ of the pixels of the raw image. An RGB image, by comparison, comprises 800x800x3 pixels. It has been found that processing the four channel BGGR image, as described below, has essentially the same denoising effect but reduces the processing time, thereby resulting in at least 25% less time to process the BGGR image compared to the raw bayer pattern image. As the processing time of the raw image is less than the processing time of a corresponding RGB image, the improvement also results in at least 25% less time to process the BGGR image compared to the RGB image.

At 204, after receiving the images 202a the processing instructions may, optionally, demosaic the images, forming demosaiced images 204a. The process to demosaic, also known as color reconstruction, is a conversion from Bayer level color space to RGB color space and is a prior-art digital image processing algorithm used to reconstruct a full color image from incomplete color samples output from an image sensor overlaid with a color filter array (CFA), such as a Bayer filter. A Freeman interpolation (described in U.S. Pat. No. 4,774,565) may be made in order to remove color artifacts in the image data. However, also other algorithms for demosaicing may be used.

Optionally, the images may be denoised, for example using a prior-art process, such as a Fourier transformation, to remove fixed pattern noise. Images may incorporate fixed pattern noise during acquisition, compression, and transmission, which adversely affects video processing, edge detection and other image analysis and tracking functions. However, since noise, edge, and texture are high frequency components of images, denoising could result in the loss of image details. In particular low resolution image sensors, such as 400x400 analog sensors, may show fixed pattern noise. It is also possible to find fixed pattern noise in digital image sensors.

The fixed pattern noise should be removed before generating the edge map (e.g. before processing the source image with the SDDTT) so as to produce a better edge map than the edge map that would result if the fixed pattern noise was not removed. The Fourier transformation may be used on the raw images, the RGB images, or the gamma-enhanced RGB images. In other words, the image with the fixed pattern noise may be the image received from the videoscope or an image based on the image received from the videoscope, upon which some pre-processing was performed before the fixed pattern noise is reduced. The resulting images may be source images and in any case the transformation is performed before the images are made available to the SDDTT as source images. A filter is applied to remove the noise, in the frequency domain, and then the inverse Fourier transform is applied. The processing instructions may perform additional pre-processing with prior-art processes, such as color correction, temperature correction, white balance, resizing, deshading, and green imbalance correction, as mentioned above, before the images are made available to the SDDTT as source images and post-processing thereafter. For example, a green imbalance correction block can be performed pre-processing and a color correction block can be used in post-processing before sharpening. Dark-frame subtraction and convolution-based fixed pattern noise removal techniques can be used in lieu of the Fourier transformation.

The image processing performed above is performed on images consecutively. When a raw image is demosaiced to produce RGB images, the RGB images can be referred to as "source image" for simplicity even though the R, G, and B images (channels) are processed thereafter. A color space is a mathematical model that defines a set of primary colors, which are used to create all other colors in the space. As further described below, the source image may also be processed in the YUV color space. The source image may also be processed in HSL and CMYK color spaces.

At 206, the SDDTT processes the source image in a single pass and outputs a denoise map 206a and an edge map (i.e. edge and texture) 206b. If the source image is an RGB image, the other two source images forming the RGB image set (e.g. channels) are also processed, in the same manner. The processing instructions make available source images to the SDDTT. In this context "make available" indicates storing the source images in a location known to the SDDTT so that the SDDTT can access them. The location may be a buffer or other memory. Alternatively, "make available" can mean that a pointer indicative of the location of the source images is provided to the SDDTT. The source images may be the images made available by the videoscope interface. In this context "corresponding" indicates that images may be pre-processed in prior-art processes before they are made available for use by the SDDTT. For example, the image sensor may output analog or digital signals and the signals may be conveyed to the video processor in analog or digital format. If in analog format, the processing instructions digitize the analog signals. The image data may comprise raw images. If the processing instructions demosaic the raw images, each of the red, green and blue images of each channel is considered a source image. Otherwise, the raw image is the source image. Processing raw images may reduce the processing time.

As indicated above, image sensor parameters may be provided to the SDDTT. Using the gain (and/or exposure, etc.) as an input furthermore forces the network to make the predicted residual (and the noise and edge maps) more specific to the noise level of the original input image.

At 208, the processing instructions process the source image with the denoise map. Such processing may comprise subtracting the denoise map from the source image to subtract noise. The subtraction removes noise. This results in a denoised image 208a. The noise map may comprise an image of the same size as the source image such that subtraction may be performed pixel by pixel.

At 210, the processing instructions, optionally, gamma correct the images to form gamma-corrected images 210a. Gamma correction is a prior-art process used to encode and decode luminance or tristimulus values in video or still image systems. Gamma corrections take advantage of the non-linear manner in which humans perceive light and color to optimize the usage of bits of images. The human perception of brightness has greater sensitivity to relative differences between darker tones than between lighter tones. If images are not gamma-encoded, they allocate too many bits to highlights that humans cannot differentiate and too few bits to shadow values that humans are sensitive to and would require more bits/bandwidth to maintain the same visual quality. Examples of non-linear gamma correction techniques are provided in U.S. Patent No. 11,096,553. Gamma correction increases the dynamic range of images and therefore improves the ability of subsequent processing to detect edges and features.

It may be desirable to include multiple non-linear gamma correction models in memory and to use different models with different videoscopes. Using the non-linear gamma correction model has the effect that a first pixel having a low pixel intensity value, though above zero, is significantly scaled up, that is, the low pixel intensity value is significantly increased, while a second pixel having mid-range pixel intensity value is only slightly adjusted or not adjusted at all, and a third pixel having a high pixel intensity, is scaled down, that is, the high pixel intensity value is decreased. Different models can be used to compensate for variations in body lumens, including shape, color, and light reflecting properties. For example, the color and surface texture of a bronchial tube differs from the color and surface texture of the colon. Different models can be used for different image sensor types. Different models can be used for different combinations of endoscopic procedures and image sensor type, e.g. different models may be used for the same image sensor type when used in different procedures. A non-linear gamma correction model suited to a specific lumen and illumination intensity of the light emitters of the videoscope may be used. The video processor may select the model from a group of models based on the connection to the videoscope or in any other manner, as described above with reference to selection of a specific SDDTT.

At 212, the processing instructions process the gamma-corrected or the denoised image, or a variation thereof, with the edge map. Such processing may comprise adding the edge map to the denoised image to generate the near real-view image 212a. It may be appropriate to re-size the image before adding the scaled edge map to the denoised image. The process has run at less than 30 milliseconds per image, even at less than 24 milliseconds per image, in a portable image processor.

Obtaining both the denoise map and the edge map from a single neural network, potentially in a single pass, reduces computational time compared to using two separate neural networks, one for each task. Arranging a neural network with both a denoise output block and an edge output block decorrelates the edge/texture information from the noise to produce better results compared to using two neural networks. In some instances, the sharpness level of the image enhanced by traditional methods, such as averaging noise and deblurring edge detection techniques, produced a sharpness effect improvement of about 2.5. The present method, by contrast, produced a sharpness effect greater than 5.0, in instances about 10.0 with images from a low resolution sensor, such as 400x400 resolution sensor. The improvement attributable to the synergistic result of simultaneously reducing noise and increasing sharpness with the trained model. Image sharpness can be measured by the "rise distance" of an edge within the image. With this technique, sharpness can be determined by the distance of a pixel level between 10% to 90% of its final value (also called 10-90% rise distance). Another way to determine the sharpness effect improvement is to use a sharpness block in the processing pipeline - most pipelines use one after a denoising block. Sharpness blocks use a sharpness value corresponding to the amount of desired sharpness increase. As the sharpness value increases the image becomes pixilated and artificial and the noise also increases. U.S. Patent No. 11,328,390 offers a user the possibility to choose a value that provides a desired balance between sharpness and noise.

Processing images in pipelines with and without the trained model to achieve the same sharpness would allow a user to see what the sharpness block values are that achieve equivalent results. In one example observed as explained herein, without the trained model the sharpness value was 2.5 and by using the trained model the sharpness block value could be raised to 7.5, 10.0 and perhaps even higher. The edge map allows visual enhancement of even smaller tissue structures and with more specificity than with conventional image processing methods. This is due in part to extracting ground-truth edges from ground-truth scene images that contain less noise, enabling extraction of more reliable edge/texture information in the images. In one example, the dataset used to train the network may comprise between 1000 and 5000 images. The dataset can, of course, include more than 5000 images. The image dataset may comprise scenes with patterns and scenes without patterns. The scenes may be devoid of human or animal tissue. The scenes may comprise cloth or other materials with and without patterns, captured at two or more gain levels. The image dataset may comprise scenes containing cloth, prints, protein, vegetables, leaves, preserved human or animal tissue, and objects. Objects can be modeled, such as with clay, play-doh, acrylonitrile butadiene styrene (ABS), resin, and other polymers. The variety of scenes are used to increase textures, color variations, and stuctural features that the network can learn from. Each of these can be considered a variable, in aggregate forming a multivariate dataset.

In one example, the image dataset is obtained by setting multivariate scenes and capturing images of the multivariate scenes with different image sensor gain values (and/or different exposure values and/or exposure/gain combinations) while the scenes are immobile. Additional details of this methodology are provided below. In this example, an endoscope with a desired image sensor is positioned to capture each of the multivariate scenes multiple times for each of the gain values. Thus, a first of M scenes is captured with a first gain value N times while the endoscope is immobile relative to the scene. Subsequently, the first scene is captured with different gain values, N times for each gain value, without moving the endoscope. The gain values are, preferably, equally spaced over the range of gain values. The image processor may transmit to the endoscope the gain values. If the gain values are normalized over the gain range of the image sensor, the gain values may be 0, 0.5, and 1.0, for example. The normalized gain values may also be 0, 0.25, 0.50, 0.75, and 1.00. Other variations providing multiple gain values are of course possible. The normalized gain values may also be 0 and 1.00, e.g. two values. The gain values should correspond to gain values likely to be used during endoscopic procedures. As explained below, the actual gain values used to generate images during the endoscopic procedure are input to the trained model, therefore training the model with the same gain values may result in better training.

After capturing images of the first scene, a second of the M scenes is created and imaged in the same manner. The process is repeated until the M scenes are imaged. The gain values do not need to be identical for each scene but, preferably, they are. It is however possible to image some scenes at multiple gain values and other scenes with fewer gain values. The object is to provide sufficient variation to the model for training purposes without creating an impractically large dataset. Some scenes may be captured with five gain values and others with three, for example, to sample enough variation without ineffective sampling. Generally, the gain values should capture the edges of the range and the center, with some scenes being captured with gain values intermediate the edges and center. The same process can be expanded by imaging the scenes at different exposures, and at different combinations of exposure and gain. It can be seen, therefore, that the number of training images can grow to an impractical number, therefore imaging some scenes more than others can balance training value and practicality/training costs. The M scenes can include scenes with patterns and scenes without patterns. The scenes may be devoid of human or animal tissue. The image dataset may comprise scenes containing cloth, prints, protein, vegetables, leaves, preserved human or animal tissue, and objects. Objects can be modeled, such as with clay, play-doh, ABS resin, and other polymers. The variety of scenes are used to increase textures, color variations, and structural features that the network can learn from.

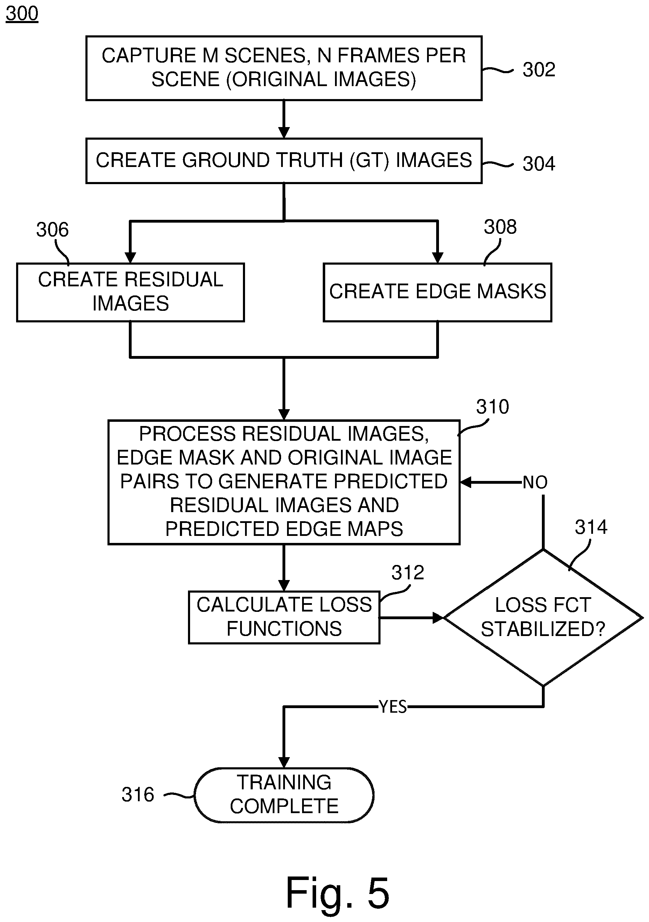

FIG. 5 presents a flowchart 300 of an embodiment of the method to train the neural network. Because noise is dependent on the image sensor, it is preferable to train the neural network with images obtained with an image sensor type and then using the resulting trained model with videoscopes having the same sensor type, as indicated above. The sensor type is, preferably, a sensor of the same model as the sensor used in training. Matching the image sensor and the model in this manner may produce better results than using the trained model on a different sensor type. However, models may vary for reasons unrelated to the noise the sensor may produce, therefore similar models may use the same sensor type. Alternatively, the sensor type represents the technology of the image sensor, such as back-side illumination, resolution, dynamic range, signal-to-noise ratio and low-light sensitivity. More broadly, a sensor may be of the same type if it produces the same type of noise. This can be determined empirically by collecting images of test patches under controlled lighting conditions with different sensors and comparing the noise characteristics, such as the presence of fixed pattern noise and signal-to-noise ratio for similar resolutions. If the characteristics are similar, for example +/- 20% of each other, the sensors can be considered to be of the same type.

The method begins, at 302, with capturing N images from each of M scenes. This results in NxM images, referred to as "original" images. The original images are captured with a desired image sensor/videoscope, thereby the trained model will produce optimal results when used with the desired videoscope. The conditions surrounding the scenes should be very stable. Scene movement, or movement between the image sensor and the scene, or changes in lighting, can cause the images to lose information necessary for subsequent edge detection. It is also important for denoising, because movement will cause edge details to be included in the residual noise images, which will cause the network to remove such structures resulting in a blurry denoised image. Furthermore, the image sensor gain G(m) for each scene is saved to allow making the trained model gain dependent as the noise characteristics change as a function of the gain. N may be greater than 15, less than 900, and potentially than 1200. A larger number increase the quality of the results at a cost of diminishing returns. The M scenes do not require patient data, the scenes can contain generic views. As described above, the scenes should provide variation in color, height, and, generally, different features at different levels so that the network can learn to distinguish the features in the training image dataset. The amount of noise reduced for GT images depends on the square root of number of images averaged. It is therefore possible to calculate N based on how much noise it is desired to reduce, e.g. averaging four images cuts the magnitude of the noise in half. The images captured for at least some scenes should also have variations in image sensor gain, exposure, and both gain and exposure pairs. Preferably, the variations are approximately equally distributed over the entire dataset.

The network can be trained with raw images, RGGB images (4 channels off the raw images) and also with RGB images. Optionally, raw images are demosaiced to produce RGB images before the method continues.

The method continues, at 304, with creating a ground-truth (GT) images from the original images. The GT images may be created by averaging the original N images from each scene to create M GT images.

At 306, residual images are created by subtracting from each original image of a scene the corresponding GT image (of the particular scene) to characterize the noise in each original image. This results in NxM residual images. The residual image is unique for each original image. This also results in NxM residual/original image pairs.

At 308, an edge/texture mask is extracted from each of the GT images. This results in M edge/texture masks. The edge/texture mask is the same for all original images from the same scene.

The edge/texture mask can be obtained with any prior-art edge detection algorithm. For example, the edge/texture masks can be obtained by filtering a GT image and then subtracting the filtered image from the GT image to obtain the edge/texture content. Example filters include Canny, Laplacian, Prewitt, Scharr, and Sobel, although other known filters can also be used. Filters are used to emphasize the edges and the edge transitions in an image. As an example, the Canny filter is a multi-stage edge detector that computes the intensity of pixel gradients. Potential edges are thinned down to 1-pixel curves by removing non-maximum pixels of the gradient magnitude. Edge pixels with a gradient value below a threshold are removed, and what is left are the components of the edge mask. In another example, an original image is filtered with a Gaussian filter and the output is subtracted from the original image. The pixel to pixel differences define edge content. The difference image is scaled (multiplied by factor) and added back to the original image to result in a sharper image. This process can be performed with RGB images as well as the Y-channel of a YUV image. The Y-channel sharpening is more efficient because it is performed on one channel vs. three channels for RGB images. Color space conversions are performed using prior-art methods. Generally, conversions between color spaces comprise matching colors and describing, using the parameters of each color space, the matched colors. To convert an image, the values in one color space, the source color space, for each pixel are converted to the values of the destination color space that match the color. The colors are mathematically represented by vectors and the color conversion then comprises multiplying the vectors by a transformation matrix corresponding to the destination color space.

At 310, the original images and the gain for each corresponding scene are processed by the neural network to generate NxM predicted residual images and NxM predicted edge images. The input gain is an optional input that will allow the network to learn how the noise changes as a function of the gain and vary the predictions accordingly. The neural network will produce two outputs: a predicted residual image and a predicted edge/texture map. In a variation of the present embodiment, the gain is not used or the same gain value is used for all images.

At 312, a two-term loss function is calculated. The first term is to measure the difference of the predicted residuals and the residual images obtained at 306. The second term is to measure the difference of the predicted edge map and the edge/texture mask obtained at 308. The differences are the errors used in a loss function. The loss function may be the mean-squared-error (MSE) of the differences. Other loss functions may be used. Example loss functions include, in addition to MSE, mean absolute error, smooth mean absolute error, etc. Plotting the errors will show that the error decreases as the model improves. Eventually the curve stabilizes, at which time the errors are substantially constant (the curve flattens). When the curve flattens the difference from one error to the next is less than a small threshold.

The purpose of the error value is to give the network a metric to improve. The network can be seen as a multitude of mathematical functions connected end-to-end in series and parallel. Each function may be a derivative function with attributes. During an epoch, the network uses the parameters, such as biases and weights. The learning rate is used in gradient descend to determine how much parameters have to be changed as a function of the loss magnitude. During an epoch, the network uses the biases to calculate output values between layers and those values pass forward to subsequent functions/neurons/layers. If the learning rate is small, the network may converge slowly and thus require many epochs to converge to a reasonably small loss-function value. If the learning rate is large, the network might not converge. The error value is used to change the network parameters so that the network will finish training, e.g. converge to a reasonably small loss-function value, in a reasonable time. An optimizer is an algorithm used to change the parameters by back-propagation. There are many prior-art optimizers. A gradient descent optimizer is preferred. The optimizer knows (can access) the layers of the network and modifies the parameters based on the error values provided to it during the training phase after each epoch.

At 314, if all the images were used (first epoch) and the curve (i.e. loss function) is not stabilized, the images are randomized and run through the neural network again in second and additional epochs, until the curve is stabilized.

Training is complete, at 316, when the difference from one error to the next is less than a small threshold, indicating that the loss function is stabilized.

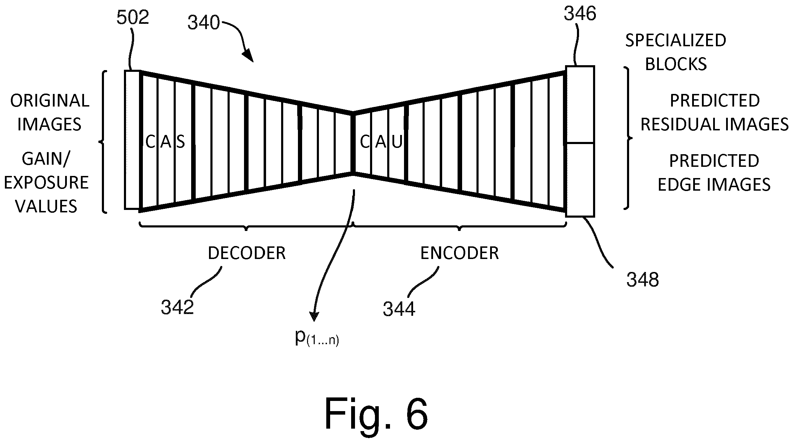

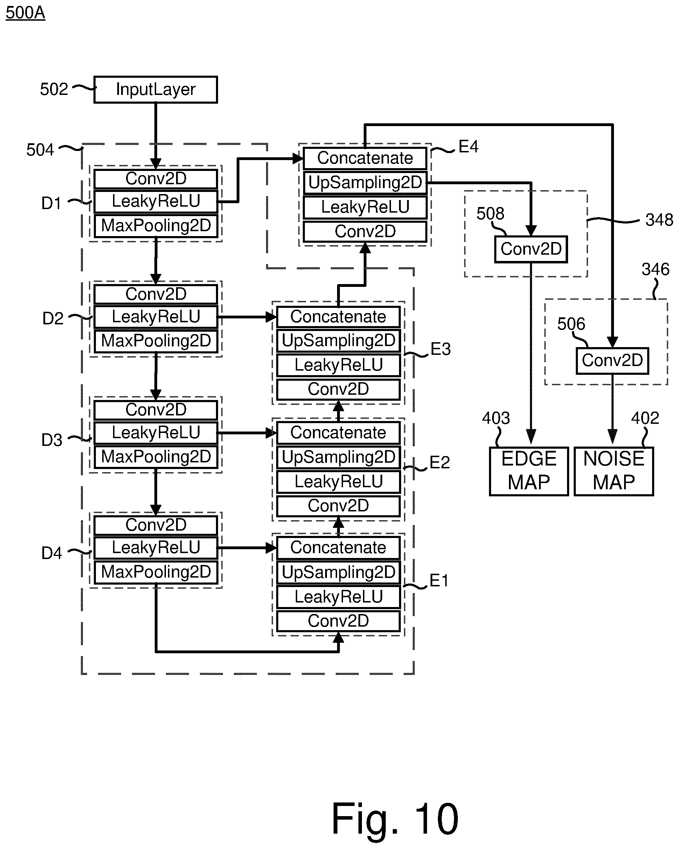

An embodiment of a neural network architecture 340 configured to generate the SDDTT is presented in FIG. 6. The architecture 340 comprises a decoder 342, an encoder 344, an input layer 502, a first specialized block 346, and a second specialized block 348. The decoder 342 comprises several processing layers, each comprising a convolution (C), an activation (A) and a subsampling (S) layer. Subsampling provides more abstraction. For example, the subsampling layers may be 800x800, then 400x400, then 200x200. The subsampling layers are equivalent. Four blocks have been determined to provide a good balance between image quality and processing cost. Of course, more blocks may be used but that requires more processing time, which might desirable when more/faster processing capacity is available. The encoder 344 comprises several processing layers, each comprising a convolution (C), an activation (A) and a upsampling (U) layer. Two specialized blocks, 346, 346, output the predicted residual images and a predicted edge/texture maps. As the neural network detects features, it builds a set of parameters p₍₁₋ₙ₎, where n is the total number of parameters. Several million parameters may be characterized via training. The parameters and architecture comprise SDDTT. As shown in FIGS. 10-12, skip connections are used as well.

The architecture uses a convolutional neural network (CNN). Optionally, a neural processing unit (NPU) or vision accelerators may be provided to improve robustness and reduce latency. Such NPU devices include, for example, Neural compute stick or NCS (Intel), Jetson Al edge devices (Nvidia), Apple neural engine (Apple), Coral Edge TPU (Google), and Neural processing engine (Qualcomm). Efficient feature detection architectures use models based on MobileNEt, ShuffleNet, or GhostNet. Feature detectors optimized for GPU computing commonly use ResNet, DarkNet, or DLA architectures.

To use the architecture the algorithm and necessary libraries are first downloaded and installed. This installs the neural network architecture. The architecture is then programmed with the location where to read the image files, and the gain values, and the location where to store the outputs. Any number of network programming languages are available. The examples shown in FIGS. 10-12 show variations of models tested to achieve the benefits described above using functions provided in Keras, which is a commonly used modeling infrastructure. Some of the functions used include Leaky ReLU, UpSampling2D, MaxPooling2D, Conv2D, and Concatenate. Leaky ReLU is a rectified linear unit activation function with a small slope for negative values instead of a flat slope. Leaky ReLUs allow a small, positive gradient when the unit is not active, helping to mitigate a vanishing gradient problem. Other activation functions may be used, beside LeakyReLU, but LeakyReLU has shown good performance. UpSampling2D is an upsampling layer for 2D inputs. UpSampling2D could be substituded with transpose convolutions. MaxPooling2D downsamples the input along its spatial dimensions (height and width) by taking the maximum value over an input window (of size defined by pool_size) for each channel of the input. The window is shifted by strides along each dimension. Conv2D is a 2D convolution layer (e.g. spatial convolution over images). This layer creates a convolution kernel that is convolved with the layer input to produce a tensor of outputs. Other convolutional layers (e.g. depth, separable) may be used. Concatenate takes as input a list of tensors, all of the same shape except for the concatenation axis, and returns a single tensor that is the concatenation of all inputs. These functions provide a convolutional neural network architecture. Other environments providing similar functionality include Tensorflow and Pytorch.

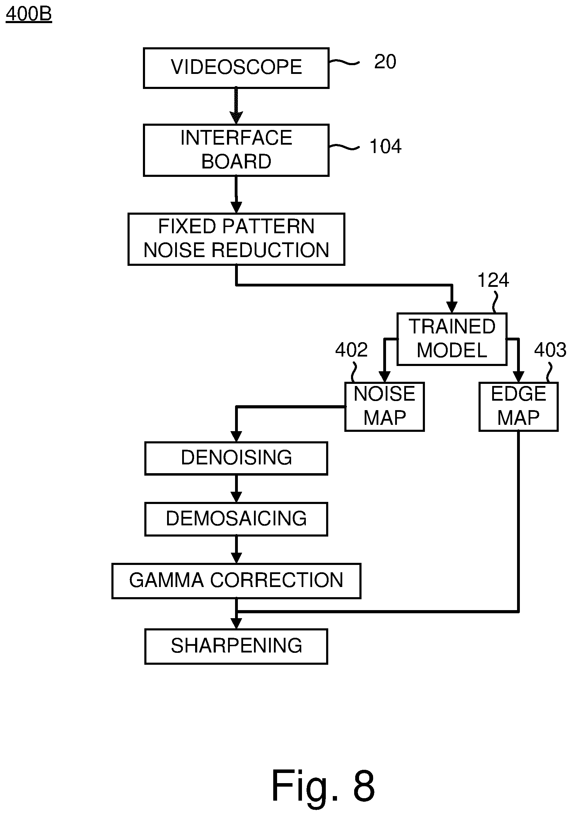

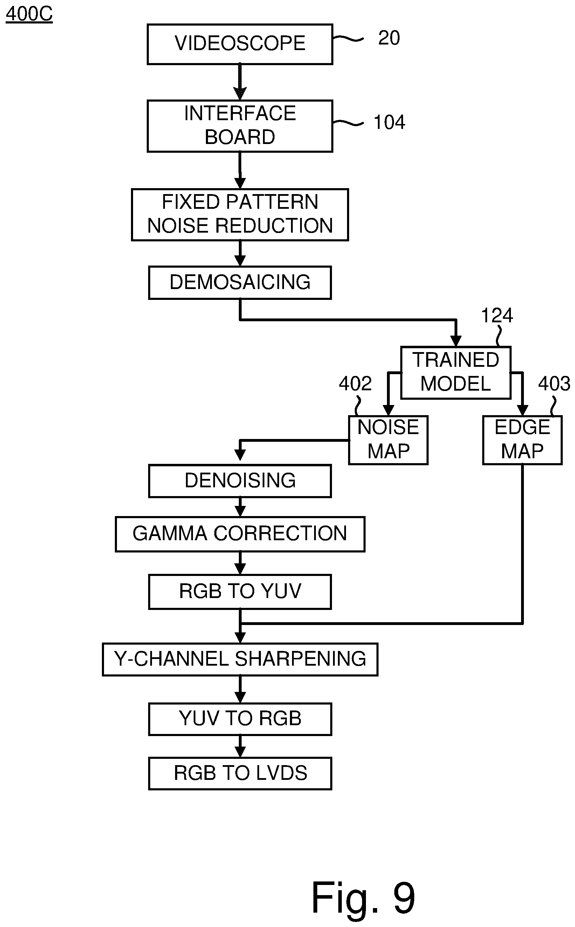

Variations of the embodiment discussed with reference to FIG. 4 will now be described with reference to FIGS. 7 to 9, showing flowcharts 400A-C. The flowcharts show the functions of a visualization system comprising a videoscope 20' and an image processing circuit 100 of the image processor 50. Common aspects will be described first, the features of each flowchart will be described in turn.

The videoscope 20' and the image processing circuit 100 may be the same as described with reference to FIGS. 1-3 and variations thereof described hereinabove. The video processor comprises the videoscope interface 104 configured to receive image data from the videoscope 20' and the processing instructions 115 including the SDDTT 124. The SDDTT 124, or trained model, can be configured in many ways. Three example configurations expanding on the description provided with reference to FIG. 6 are shown in FIGS. 10-12.

Prior to execution, the videoscope 20' is connected to the videoscope interface 104. The image processing circuit 100 determines the type of image sensor of the videoscope connected to it, as described above, and either configures the image pipeline accordingly or selects an image pipeline, from two or more image pipelines, configured to process images from said image sensor type. Configuring the image pipeline may comprise including or excluding processing blocks. For example, if the image sensor is an analog image sensor, such configuration may include processing the images to reduce or eliminate fixed pattern noise. If the image sensor is a high quality digital sensor, configuring the image pipeline may comprise bypassing the fixed pattern noise reduction processing block. Of course, a simpler image processing circuit 100 can be provided in which such configuration is not needed. For example, an image processing circuit 100 may be provided that is compatible with one image sensor type, and another image processing circuit 100 may be provided that is compatible with a different image sensor type. In another example of the present variation, the image processing circuit 100 has two or more image pipelines and configures one or more of the two or more image pipelines. In a further example of the present variation, the image processing circuit 100 has two or more image pipelines and processes images from the connected videoscope using processing blocks from the two or more pipelines to reduce the overall processing time.

The image pipeline can be processed in the FPGA 114 or by the GPU responsive to the processing instructions 115 stored memory, or a combination of both.

The images that become the source images may be pre-processed (before or after demosaicing). Pre-processing is as described above.

The image pipeline includes an optional fixed pattern noise reduction block. The fixed pattern noise reduction block may be omitted or bypassed if there is no fixed pattern noise associated with the connected videoscope.

After processing as shown in the flowcharts, the sharpened images may be converted to an LVDS format. The LVDS format is a prior-art signal format to transfer data within circuits. Eventually the LVDS signals will be converted to a signal suitable to a connected display, as is known from prior-art.

In one example, a gain value may be provided to the trained model 124 with or for each source image. The trained model is trained with gain values. The gain value represents image sensor gain. The gain value can be embedded in the source image, in which case the processing instructions extract the gain value from the source image by reading the specific pixel location and then provide the gain value as an input to the input layer of the trained model 124. The gain value can be, for example, embedded in the image by the image sensor or read by the image processing circuit from the image sensor. Alternatively, the gain value may be a fixed value corresponding to the image sensor type, such as a mid-range value of the gain range of the image sensor type. In another example, the gain value is calculated from a few images in a moving window, such as 3-5 images, which provides a degree of stabilization of the gain value. The calculation could be an average of the gain values of the images in the moving window.

In another example, a gain value is not provided to the trained model 124 because the trained model is not trained with gain values.

In another example, an exposure value is provided to the trained model 124 with or for each source image. In this example the trained model is trained with exposure values. The exposure value represents image sensor exposure. The exposure value can be embedded in the source image, in which case the processing instructions extract the exposure value from the source image by reading the specific pixel location and then provide the exposure value as an input to the input layer of the trained model 124. The exposure value can be, for example, embedded in the image by the image sensor or read by the image processing circuit from the image sensor. The exposure value can be obtained from one parameter or more than one, such as shutter speed, aperture and ISO sensitivity. If it is derived from more than one parameter, the image sensor or the image processing circuit may calculate one value in a known manner, using the exposure triangle.

In another example, an exposure value is not provided to the trained model 124 because the trained model is not trained with exposure values.

In a further example, an exposure value and a gain value are provided to the trained model 124 with or for each source image. The trained model 124 is trained with exposure and gain values.

The SDDTT comprises an encoder and a decoder. Generally, the encoder comprises three or more blocks, each block comprising a convolution (C) layer, an activation (A) layer, and a subsampling (S) layer, the convolution layer comprising trainable parameters. A batch normalization layer could be included in the block. A block could also include multiple convolution and activation layers in CACAS or CACACAS arrangement. Four blocks have provided a good balance between speed and quality. With more training or faster or more complex controllers it may be possible to use only three blocks. Five or six blocks (having the same layers) may also be beneficial to improve quality. With faster controllers the improved quality may be obtained within the timing constraints. Examples of the SDDTT are provided in FIGS. 10-12.

Referring now to a variation of the method, depicted in the flowchart 400A, the image pipeline includes a demosaicing block. The demosaicing block demosaices images corresponding to the images received by the interface 104, converting them to RGB images 204a.

The RGB images, or images corresponding to the RGB images (referred to as source images), are then provided by the processing instructions to the trained model 124. The trained model, or SDDTT 124, processes the images and outputs, in a single pass, a noise map 206a, 402 and an edge map 206b, 403. As shown, the trained model 124 resides outside the FPGA 114. However, the trained model can also be included in the FPGA 114.

In the present variation, the image pipeline includes a denoising block. The denoising block denoises the source images with the noise map 206a/402 to produce denoised images 208a. Denoising may comprise subtracting the noise map from the source images.

In the present variation, the image pipeline includes an RGB gamma correction block. Because RGB images were provided to the trained model, a gamma correction block configured to process RGB images is provided. If raw images were provided, a different gamma correction block would be used.

In the present variation, the image pipeline includes a sharpening block. The sharpening block adds (or strengthens existing) features to(of) the denoised images or images corresponding to the denoised images. Sharpening may comprise adding the edge map 403 to the gamma-corrected images.

Another variation of the method, depicted in the flowchart 400B, will now be described. The present variation is a variation of the method described with reference to FIGS. 4 and 7. The present variation of the method is the same as the variation depicted in the flowchart 400A except that the source images, supplied to the trained model 124, are raw images. The demosaicing block is placed between the denoising and the gamma correction blocks. After the images are processed by the trained model 124 and denoised, as described with reference to FIG. 7, then the images are demosaiced. Therefore, gamma correction and sharpening are performed as previously described, in the RGB color space. The model is trained with raw images. In one example, the source images, supplied to the trained model 124, are raw images but in the RGGB format. After the images are processed by the trained model 124 they are denoised, as described with reference to FIG. 7. Then the images may be demosaiced. The the denoised RGGB images may be demosaiced or they may be y may be reformatted into a single raw channel and then demosaiced.

Another variation of the method, depicted in the flowchart 400C, will now be described. The present variation is a variation of the method described with reference to FIGS. 4 and 7. The present variation of the method is the same as the variation depicted in the flowchart 400A except that, after the gamma correction block, the images are converted from the RGB to the YUV color space. Then, sharpening is performed in only the Y-channel. The images are then converted to the RGB color space. The model is trained with RGB images. Sharpening in the Y-channel is faster because only one channel is processed vs. three channels in the RGB color space.

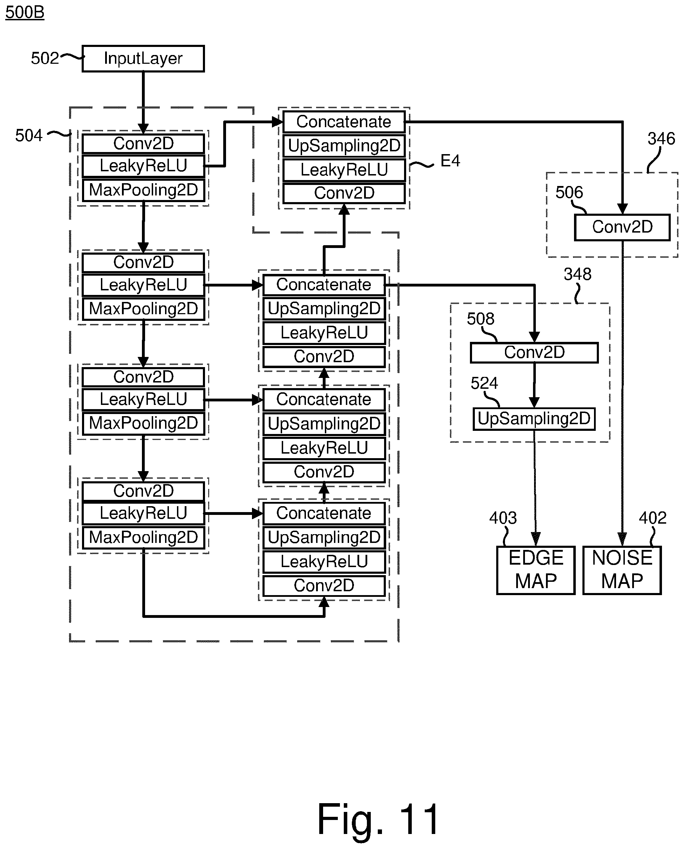

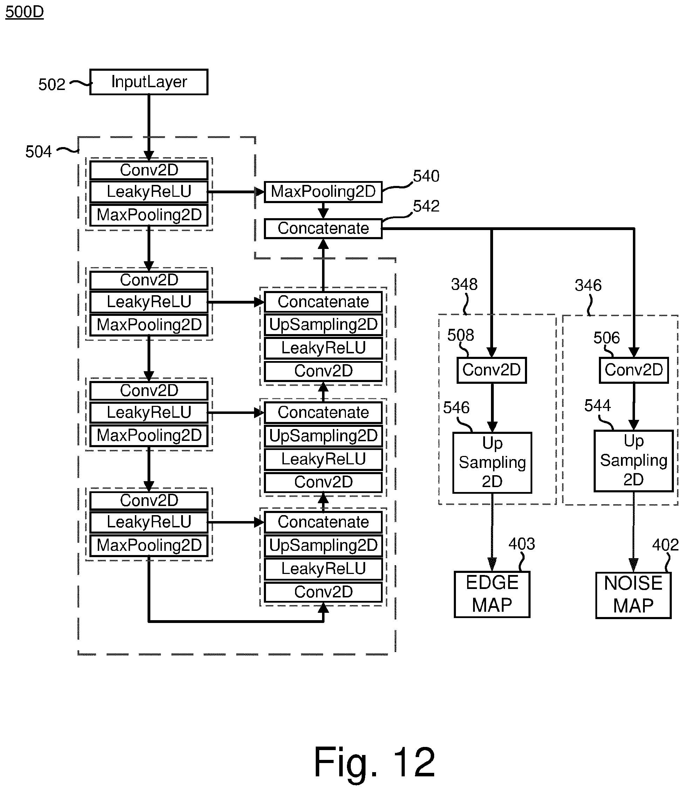

FIGS. 10 to 12 are block diagrams of configurations of the network architecture 340, depicted as 500A-500C. The network architecture 500A has an input layer 502 to which the images, gain values, etc. are provided. The network architecture 500A has four decoder blocks, D1-D4 and four encoder blocks, E1-E4. The decoder blocks and the first three encoder blocks are enclosed in a dashed outline 504. The dashed outline 504 is provided only to compact the disclosure of FIGS. 11 and 12. The configuration of the blocks inside the dashed outline 504 is the same in FIGS. 11 and 12. Each of the decoder blocks comprises the functions Conv2D, LeakyReLu, and MaxPooling2D. Each of the encoder blocks comprise the functions Conv2D, LeakyReLu, UpSampling2D, and Concatenate. Additionally, the network architecture 500A comprises skip connections from the LeakyReLu function of a decoding block to the concatenate function of an encoding block, arranged symmetrically (D1-E4, D2-E3, D3-E2, D4-E1). In other words, the SDDTT 124 comprises a decoder 342 and an encoder 344, the decoder comprising four decoder blocks D1-D4, each decoder block comprising a convolution layer, an activation layer, and a subsampling layer, each of the convolution layers comprising trainable parameters, and the encoder comprising encoder blocks including a first encoder block E1, a second encoder block E2 following the first encoder block, and a third encoder block E3 following the second encoder block, each of the encoder blocks comprising a convolution layer, an activation layer, an upsampling layer, and a concatenation layer.

The network architecture 500A has additional Conv2D layers 506 and 508 connected to the Concatenate and UpSampling2D layers, respectively, of the (fourth) encoding block, E4, which follows the third encoding block E3. These Conv2D layers can be seen as specialized blocks 346, 348 that output the noise map 402 and the edge map 403. In other words, the encoder further comprises a fourth encoder block E4 following the third encoder block, wherein the SDDTT further comprises, following the fourth encoder block, a first specialized block 346 and a second specialized block 348, the first specialized block comprising a convolution layer 506 configured to output the noise map 402, the second specialized block 348 comprising a convolution layer 508 configured to output the edge map 403, the first specialized block receiving an output from the concatenation layer of the fourth encoder block, and the second specialized block receiving an output from the upsampling layer of the fourth encoder block. The last convolutions of the network architecture 500A, denoted as 506 and 508, are performed on full resolution images.

The network architecture 500B shown in FIG. 11 is similar to the network architecture 500A except that the Conv2D layer 508 is connected to the Concatenate layer of the encoding block E3, therefore the specialized block 348 includes an UpSampling2D layer 524 that outputs the edge map 403. The last convolution for the edge map on the network architecture 500B is performed at downscaled resolution and then up-sampled. Because convolution times are very resolution dependent and costly, performing the convolution at lower resolution saves valuable processing time.

The network architecture 500C shown in FIG. 12 is similar to the network architecture 500A, with the following differences. The encoding block E4 is omitted. A MaxPooling2D layer 540 is connected to the LeakyReLu layer of the decoding block E1, and a Concatenate layer 542 is connected to the encoding block E3 and the MaxPooling2D layer 540. The Concatenate layer 542 is connected to the Conv2D layers 506, 508, each being connected to an UpSampling2D layer 544 or 546. The last convolutions for the network architecture 500C are performed at downscaled resolutions and then up-sampled, saving even more time, albeit at perhaps a slight loss in the quality of the maps.

The following items are examples of various embodiments disclosed above:
1. A method to generate near real-view images, the method comprising: by an image processing circuit (100) connected to a videoscope (20'): receiving an image (202a) from the videoscope (20'); processing a source image (204a) corresponding to the image (202a) with a single denoising and feature/edge detection trained network (SDDTT) (124), the SDDTT outputting, in a single pass, a denoise map (206a) and an edge map (206b); denoising the source image (204a) with the noise map (206a) to produce a denoised image (208a); gamma-correcting the denoised image to produce a gamma-corrected image (210a); and sharpening the gamma-corrected denoised image with the edge map (206b) to produce the near real-view image (212a).
2. The method of item 1, further comprising demosaicing the image (202a) before processing the source image (204a) with the SDDTT (124).
3. The method of item 2, further comprising, after said denoising and before said gamma-correcting the demosaiced image: color-converting the demosaiced image to a YUV color space to form a YUV image, gamma-correcting the YUV image, and after said gamma-correcting, sharpening a Y-channel of the YUV image.
4. The method of item 3, wherein sharpening the Y-channel of the YUV image comprises sharpening only the Y-channel of the YUV image, and subsequently converting the YUV image to an RGB image.
5. The method of item 3, the method further comprising, after said sharpening, color-converting the sharpened image to the RGB color space.
6. The method of item 1, wherein the source image processed by the SDDTT (124) is a raw image, the method further comprising: after said denoising and before said gamma-correcting: demosaicing the raw image.
7. The method of item 6, wherein the source image processed by the SDDTT (124) is a four channel BGGR image, each of the four channels comprising the corresponding color pixels of the raw image.
8. The method of any preceding item, wherein the videoscope (20') comprises an image sensor (42), the method further comprising, by the image processing circuit (100), determining a type or a model of the image sensor (42).
9. The method of item 8, wherein the image processing circuit (100) comprises two or more trained networks, the method further comprising, by the image processing circuit (100), selecting the SDDTT from the two or more trained networks, wherein each of the two or more trained networks was trained with images collected with a different image sensor type.
10. The method of any of the preceding items, wherein the videoscope (20') comprises a first image sensor (42) of an image sensor type, the method further comprising: capturing a first plurality of images (60) with a second image sensor (42) of the image sensor type at a first image sensor gain; capturing a second plurality of images with the second image sensor (42) at a second image sensor gain, the second image sensor gain being different than the first image sensor gain; providing the first image sensor gain and the first plurality of images to a single denoising and feature/edge detection network; processing the first plurality of images with the single denoising and feature/edge detection network to train the single denoising and feature/edge detection network; providing the second image sensor gain and the second plurality of images to the single denoising and feature/edge detection network; and processing the second plurality of images with the single denoising and feature/edge detection network to further train the single denoising and feature/edge detection network and form the SDDTT.
11. The method of any of the preceding items, wherein the videoscope (20') comprises a first image sensor (42) of an image sensor type, the method further comprising: capturing a first plurality of images (60) with a second image sensor (42) of the image sensor type at a first image sensor exposure; capturing a second plurality of images with the second image sensor (42) at a second image sensor exposure, the second image sensor exposure being different than the first image sensor exposure; providing the first image sensor exposure and the first plurality of images to a single denoising and feature/edge detection network; processing the first plurality of images with the single denoising and feature/edge detection network to train the single denoising and feature/edge detection network; providing the second image sensor exposure and the second plurality of images to the single denoising and feature/edge detection network; and processing the second plurality of images with the single denoising and feature/edge detection network to further train the single denoising and feature/edge detection network and form the SDDTT.
12. The method of any of the preceding items, wherein the videoscope (20') comprises a first image sensor (42) of an image sensor type, the method further comprising: capturing a first plurality of images (60) with a second image sensor (42) of the image sensor type at a first image sensor gain and exposure combination; capturing a second plurality of images with the second image sensor (42) at a second image sensor gain and exposure combination, the second image sensor gain and exposure combination being different than the first image sensor gain and exposure combination; providing the first image sensor gain and exposure combination and the first plurality of images to a single denoising and feature/edge detection network; processing the first plurality of images with the single denoising and feature/edge detection network to train the single denoising and feature/edge detection network; providing the second image sensor gain and exposure combination and the second plurality of images to the single denoising and feature/edge detection network; and processing the second plurality of images with the single denoising and feature/edge detection network to further train the single denoising and feature/edge detection network and form the SDDTT.
13. The method of any of items 10-12, wherein the first plurality of images and the second plurality of images comprise images of a scene.
14. The method of any of items 10-12, wherein the first plurality of images and the second plurality of images comprise images of a scene, and wherein the scene is devoid of human or animal tissue.
15. The method of any of the preceding items, the method further comprising, before processing the source image (204a) with the SDDTT (124), reducing fixed pattern noise in an image corresponding to the image (202a).
16. The method of any of the preceding items, wherein the SDDTT comprises an encoder and a decoder, the encoder comprising at least four blocks, each block comprising a convolution layer, an activation layer, and a subsampling layer, the convolution layer comprising trainable parameters.
17. The method of any of the preceding items, wherein the SDDTT 124 comprises a decoder 342 and an encoder 344, the decoder comprising four decoder blocks D1-D4, each decoder block comprising a convolution layer, an activation layer, and a subsampling layer, each of the convolution layers comprising trainable parameters, and wherein the encoder comprises encoder blocks including a first encoder block E1, a second encoder block E2 following the first encoder block, and a third encoder block E3 following the second encoder block, each of the encoder blocks comprising a convolution layer, an activation layer, an upsampling layer, and a concatenation layer, wherein the SDDTT 124 further comprises, after the third encoder block E3, a first specialized block 346 comprising a convolution layer 506 and being configured to output the noise map 402, and wherein the SDDTT 124 further comprises a second specialized block 348 comprising a convolution layer 508 and being configured to output the edge map 403.
18. The method of item 17, wherein the encoder further comprises a fourth encoder block E4 following the third encoder block, wherein the first specialized block 346 receives an output from the concatenation layer of the fourth encoder block E4, and wherein the second specialized block 348 receives an output from the upsampling layer of the fourth encoder block E4.
19. The method of item 17, wherein the encoder further comprises a fourth encoder block E4 following the third encoder block, wherein the first specialized block 346 receives an output from the concatenation layer of the fourth encoder block E4, and wherein the second specialized block 348 receives an output from the concatenation layer of the third encoder block E3.
20. The method of item 17, wherein the encoder further comprises a downsampling layer 540 and a fourth concatenation layer 542, wherein the fourth concatenation layer 542 follows the downsampling layer 540 and the third encoder block E3, wherein the first specialized block 346 receives an output from the fourth concatenation layer 540 and comprises an upsampling layer 544 following the convolution layer 506, and wherein the second specialized block 348 receives the output from the fourth concatenation layer 540 and comprises an upsampling layer 546 following the convolution layer 508.
21. The method of any of the preceding items, wherein an image dataset is obtained to train the SDDTT by setting multivariate scenes and capturing images of the multivariate scenes with different image sensor gain values (and/or different exposure values and/or exposure/gain combinations) while the scenes are immobile. The gain/exposure values are, preferably, equally spaced over the range of gain values available for a particular image sensor type.
22. The method of any of the preceding items, wherein the image processing circuit (100) comprises a controller (110) and non-volatile memory (120), the non-volatile memory (120) having embedded therein processing instructions including the SDDTT.
23. The method of any of any one of items 1-21, wherein the image processing circuit (100) comprises a controller (110) and non-volatile memory (120), the non-volatile memory (120) having embedded therein the SDDTT, and the controller (110) comprising a central processing unit and a graphics processing unit.
24. A video processor comprising: a controller (110) comprising the image processing circuit (100), the image processing circuit (100) including a non-volatile memory (120) having embedded therein processing instructions including the SDDTT, the processing instructions being configured to implement the method of any of items 1-20.
25. A visualization system comprising the videoscope (20'), a display, and the video processor of item 14, wherein the processing instructions are configured to present with the display images corresponding to the near real-view image (212a).

In all embodiments and variations thereof, videoscope interfaces may be exchangeable. Provision of exchangeable videoscope interfaces allows for adoption of evolving image sensor and endoscope technologies, thus use of existing or future-developed external video displays could allow presentation of higher resolution or otherwise improved video. Use of external video displays could also leverage existing capital investments. By exchangeable it is meant that the videoscope interfaces can be plugged and unplugged through a side of the housing without tearing into the housing or the circuits therein.

In all embodiments and variations thereof, the videoscope may be disposable and may not be intended to be cleaned and reused. Alternatively, the videoscope may be reusable.

In some variations of the present embodiment, the videoscope and the video processor comprise wireless transceivers to exchange image data and configuration data. The videoscope may comprise a battery to power the image sensor and light sources.

### Parts list:

| # | **NAME** |
|---|---|
| 10 | visualization system |
| 20 | endoscope |
| 20' | videoscope |
| 21 | handle |
| 22 | housing |
| 23 | endoscope cable |
| 24 | connector |
| 25 | steering actuator |
| 30 | insertion cord |
| 31 | insertion tube |
| 32 | bending section |
| 34 | distal tip |
| 36 | tool |
| 40 | camera assembly |
| 42 | image sensor |
| 44 | light emitters |
| 50 | video processor |
| 52 | housing |
| 54 | display |
| 60 | images |
| 62(i), | images |
| 62(i=3) | |
| 62(i)' | near real-view image |
| 100 | image processing circuit |
| 102 | cable socket |
| 104 | videoscope interface |
| 110 | controller |
| 112 | CPU |
| 114 | FPGA |
| 116 | GPU |
| 110 | controller |
| 118 | user interface |
| 120 | memory |
| 122 | GUI logic |
| 123 | GUI |
| 124 | SDDTT |
| 130 | video output board |
| 340 | neural network architecture |
| 342 | decoder |
| 344 | encoder |
| 346 | first specialized block |
| 348 | second specialized block |
| 402 | noise map |
| 403 | edge map |
| 500A-C | network architectures |
| 502 | input layer |
| 504 | outline of a portion of the network architectures |
| 506 | convolution layer |
| 508 | convolution layer |
| 524 | upsampling layer |
| 540 | downsampling layer |
| 542 | concatenation layer |
| 544 | upsampling layer |
| 546 | upsampling layer |
| D1-D4 | decoder blocks |
| E1-E4 | encoder blocks |

## Claims

1. A method to generate near real-view images, the method comprising:
by an image processing circuit (100) connected to a videoscope (20'):
receiving an image (202a) from the videoscope (20');
processing a source image (204a) corresponding to the image (202a) with a single denoising and feature/edge detection trained network (SDDTT) (124), the SDDTT outputting, in a single pass, a denoise map (206a) and an edge map (206b);
denoising the source image (204a) with the noise map (206a) to produce a denoised image (208a);
gamma-correcting the denoised image to produce a gamma-corrected image (210a); and
sharpening the gamma-corrected denoised image with the edge map (206b) to produce the near real-view image (212a).

2. The method of claim 1, further comprising demosaicing the image (202a) before processing the source image (204a) with the SDDTT (124).

3. The method of claim 2, further comprising, after said denoising and before said gamma-correcting the demosaiced image: color-converting the demosaiced image to a YUV color space to form a YUV image, gamma-correcting the YUV image, and after said gamma-correcting, sharpening a Y-channel of the YUV image.

4. The method of claim 1, wherein the source image processed by the SDDTT (124) is a raw image, the method further comprising: after said denoising and before said gamma-correcting: demosaicing the raw image.

5. The method of any preceding claim, wherein the videoscope (20') comprises an image sensor (42), the method further comprising, by the image processing circuit (100), determining a type or a model of the image sensor (42).

6. The method of claim 5, wherein the image processing circuit (100) comprises two or more trained networks, the method further comprising, by the image processing circuit (100), selecting the SDDTT from the two or more trained networks, wherein each of the two or more trained networks was trained with images collected with a different image sensor type.

7. The method of any of the preceding claims, wherein the videoscope (20') comprises a first image sensor (42) of an image sensor type, the method further comprising:
capturing a first plurality of images (60) with a second image sensor (42) of the image sensor type at a first image sensor gain;
capturing a second plurality of images with the second image sensor (42) at a second image sensor gain, the second image sensor gain being different than the first image sensor gain;
providing the first image sensor gain and the first plurality of images to a single denoising and feature/edge detection network;
processing the first plurality of images with the single denoising and feature/edge detection network to train the single denoising and feature/edge detection network;
providing the second image sensor gain and the second plurality of images to the single denoising and feature/edge detection network; and
processing the second plurality of images with the single denoising and feature/edge detection network to further train the single denoising and feature/edge detection network and form the SDDTT.

8. The method of any of the preceding claims, the method further comprising, before processing the source image (204a) with the SDDTT (124), reducing fixed pattern noise in an image corresponding to the image (202a).

9. The method of any of the preceding claims, wherein the SDDTT 124 comprises a decoder 342 and an encoder 344, the decoder comprising four decoder blocks D1-D4, each decoder block comprising a convolution layer, an activation layer, and a subsampling layer, each of the convolution layers comprising trainable parameters, and wherein the encoder comprises encoder blocks including a first encoder block E1, a second encoder block E2 following the first encoder block, and a third encoder block E3 following the second encoder block, each of the encoder blocks comprising a convolution layer, an activation layer, an upsampling layer, and a concatenation layer, wherein the SDDTT 124 further comprises, after the third encoder block E3, a first specialized block 346 comprising a convolution layer 506 and being configured to output the noise map 402, and wherein the SDDTT 124 further comprises a second specialized block 348 comprising a convolution layer 508 and being configured to output the edge map 403.

10. The method of claim 9, wherein the encoder further comprises a fourth encoder block E4 following the third encoder block, wherein the first specialized block 346 receives an output from the concatenation layer of the fourth encoder block E4, and wherein the second specialized block 348 receives an output from the upsampling layer of the fourth encoder block E4.

11. The method of claim 9, wherein the encoder further comprises a fourth encoder block E4 following the third encoder block, wherein the first specialized block 346 receives an output from the concatenation layer of the fourth encoder block E4, and wherein the second specialized block 348 receives an output from the concatenation layer of the third encoder block E3.

12. The method of claim 9, wherein the encoder further comprises a downsampling layer 540 and a fourth concatenation layer 542, wherein the fourth concatenation layer 542 follows the downsampling layer 540 and the third encoder block E3, wherein the first specialized block 346 receives an output from the fourth concatenation layer 540 and comprises an upsampling layer 544 following the convolution layer 506, and wherein the second specialized block 348 receives the output from the fourth concatenation layer 540 and comprises an upsampling layer 546 following the convolution layer 508.

13. The method of any of any one of claims 1-12, wherein the image processing circuit (100) comprises a controller (110) and non-volatile memory (120), the non-volatile memory (120) having embedded therein the SDDTT, and the controller (110) comprising a central processing unit and a graphics processing unit.

14. A video processor comprising:
a controller (110) comprising the image processing circuit (100), the
image processing circuit (100) including a non-volatile memory (120) having embedded therein processing instructions including the SDDTT, the processing instructions being configured to implement the method of any of claims 1-12.

15. A visualization system comprising the videoscope (20'), a display, and the video processor of claim 14, wherein the processing instructions are configured to present with the display images corresponding to the near real-view image (212a).
